(19) Europäisches Patentamt
European Patent Office
Office européen des brevets

(11) Numéro de publication: **0 161 156**
**B1**

(12) **FASCICULE DE BREVET EUROPÉEN**

(45) Date de publication du fascicule du brevet: **15.02.89**

(51) Int. Cl.⁴: **C 07 C 63/66**, C 07 C 69/773, A 61 K 31/19, C 07 C 103/76

(21) Numéro de dépôt: **85400667.3**

(22) Date de dépôt: **03.04.85**

(54) **Nouveaux dérivés de l'acide vinyl-4 benzoïque, leur procédé de préparation et leurs applications en thérapeutique et comme ligands.**

(30) Priorité: **06.04.84 FR 8405531**

(43) Date de publication de la demande: **13.11.85 Bulletin 85/46**

(45) Mention de la délivrance du brevet: **15.02.89 Bulletin 89/7**

(84) Etats contractants désignés: **AT BE CH DE FR GB IT LI LU NL SE**

(56) Documents cité:
**EP-A-0 084 667**
**DE-A-2 427 404**
**FR-A-2 117 470**
**FR-A-2 422 620**
**US-E-27 031**

**Le dossier contient des informations techniques présentées postérieurement au dépôt de la demande et ne figurant pas dans le présent fascicule.**

(73) Titulaire: **LABORATOIRES CHAUVIN S.A., 104 Rue de la Galéra, F-34000 Montpellier (FR)**

(72) Inventeur: **Coquelet, Claude, 113 rue des Lilas, F-34980 St Gely- du- Fesc (FR)**
Inventeur: **Roussillon, Samia, La Boissière, F-34150 Gignac (FR)**
Inventeur: **Sincholle, Daniel, 343 avenue de la trémoulette, F-34980 St- Clement- La- Riviere (FR)**
Inventeur: **Bonne, Claude, 12bis avenue Aristide Briand, F-94360 Bry- Sur- Marne (FR)**
Inventeur: **Alazet, Alain, 5 Impasse des Vignerons, F-34300 Agde (FR)**

(74) Mandataire: **Polus, Camille, c/o Cabinet Lavoix 2, Place d'Estienne d'Orves, F-75441 Paris Cedex 09 (FR)**

**EP 0 161 156 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne de nouveaux dérivés de l'acide vinyl-4 benzoïque qui ont une activité sur la croissance et la différenciation des tissus biologiques et qui peuvent être utilisés en thérapeutique pour le traitement de l'acné, du psoriasis, des troubles de la kératinisation, dans certaines formes de cancer et comme agents cicatrisants, notamment dans le domaine oculaire.

La vitamine A est essentielle à certaines fonctions telles que la vision, la croissance et la reproduction. Elle joue un rôle important dans le contrôle de la croissance et de la différenciation des tissus épithéliaux. La carence en vitamine A provoque une hyperkératose cutanée et une métaplasie kératinisante des muqueuses.

Bien que le mode d'action des rétinoïdes (analogues de la vitamine A) soit encore méconnu, il a été montré:

- qu'ils ralentissaient la croissance et la progression des cellules précancéreuses et cancéreuses;
- qu'ils possédaient un effet anti-inflammatoire et pouvaient intervenir dans le mécanisme de la cicatrisation en agissant au niveau de la synthèse de protéines indispensables au processus de réparation tissulaire.

Les rétinoïdes sont utilisés comme agents thérapeutiques pour le traitement de l'acné et du psoriasis graves, ainsi que des troubles de la kératinisation. Cependant, bien que très efficaces,ces produits ont l'inconvénient majeur de présenter des effets secondaires non négligeables: hypervitaminose A, toxicité, irritations.

Dans FR-A-2 422 620, on a décrit des dérivés du stilbène présentant une certaine analogie de structure et d'activité avec les rétinoïdes.

La présente invention vise à fournir de nouveaux composés qui se lient au récepteur de l'acide rétinoïque (C.R.A.B.P.) au niveau tissulaire et présentent un intérat thérapeutique similaire aux rétinoïdes sans en avoir les effets toxiques, notamment dans le domaine oculaire.

La présente invention a ainsi pour objet des composés de formule (I)

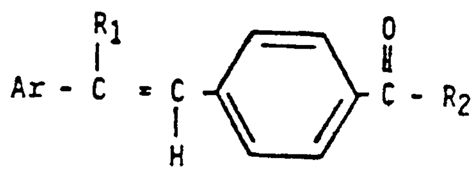

(I)

dans laquelle:

$R^1$ représente un groupe mèthyle;

Ar représente un groupe phényle, un groupe phényle substitué par un groupe alkyle en $C_1$-$C_4$, un groupe hétéroaromatique monocyclique contenant un ou deux hétéroatomes choisis parmi l'azote l'oxygène et le soufre et ayant de 5 à 11 chaînons, ou un tel groupe hétéroaromatique substitué par un groupe alkyle en $C_1$-$C_4$;

$R^2$ représente un groupe hydroxy; un groupe aminé de formule

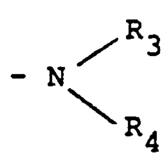

dans laquelle $R_3$ et $R_4$ représentent, indépendamment l'un de l'autre, un atome d'hydrogène, un groupe alkyle en $C_1$-$C_6$ ou amino(alkyle en $C_1$-$C_2$) ou bien $R_3$ et $R_4$ forment, avec l'atome d'azote sur lequel ils sont fixés, un groupe hétérocyclique saturé ou non ayant de 5 à 8 chaînons et pouvant contenir un autre hétéroatome choisi parmi l'azote, l'oxygène et le soufre; un groupe alkoxy en $C_1$-$C_4$; un groupe de formule -O-Ar, Ar ayant la signification donnée ci-dessus; un groupe aminoalkoxy de formule

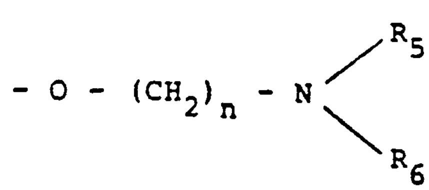

dans laquelle $R_5$ et $R_6$ représentent, indépendamment l'un de l'autre, un groupe alkyle en $C_1$-$C_4$ et n = 1 à 4; ainsi que leurs sels pharmaceutiquement acceptables.

La présente invention a également pour objet des compositions thérapeutiques contenant, à titre de principe actif, un composé de formule (I) ou l'un de ses sels pharmaceutiquement acceptables.

Par sels pharmaceutiquement acceptables, on désigne les sels d'addition que forment les composés de

formule (I) à groupe basique avec des acides pharmaceutiquement acceptables, ainsi que les sels que forment les composés de formule (I) à groupe acide avec des bases pharmaceutiquement acceptables.

Les "sels d'addition avec des acides pharmaceutiquement acceptables" désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; des sels métalliques acides, tels que l'orthophosphate disodique et le sulfate monopotassique, et des acides organiques, tels que l'acide formique, l'acide acétique, l'acide propionique, l'acide glycolique, l'acide oxalique, l'acide fumarique, l'acide lactique, l'acide succinique, l'acide tartrique et l'acide pamoïque.

De même, "les sels avec des bases pharmaceutiquant acceptables" désignent les sels qui ne modifient pas les propriétés biologiques des acides libres. Ces sels peuvent être notamment ceux formés avec des bases minérales, telles que l'hydroxyde de sodium, l'hydroxyde de potassium, l'hydroxyde de lithium, l'hydroxyde de calcium, l'hydroxyde de magnésium, ou des bases organiques telles que la glucamine, la N-méthyl-glucamine, la N,N-diméthylglucamine, l'éthanolamine, la diéthanolamine, la morpholine, la N-méthyl morpholine et la tris-(hydroxyméthyl)-méthylamine.

Une classe préférée de composés de formule (I) est celle formée par les composés ayant une configuration E de la molécule par rapport à la double liaison.

Les composés de formule (I) peuvent être préparés, d'une manière générale, par réaction de Wittig d'un composé de formule (II)

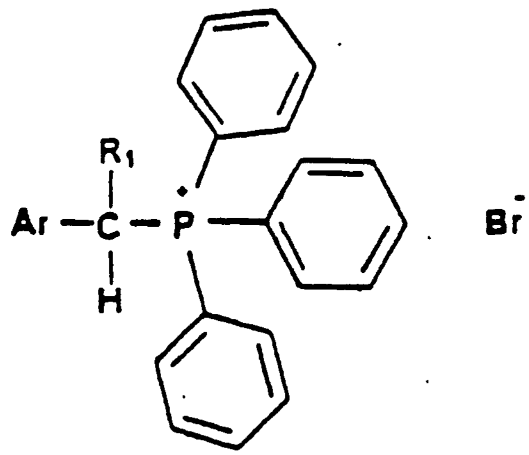

(II)

avec un benzaldéhyde de formule (III)

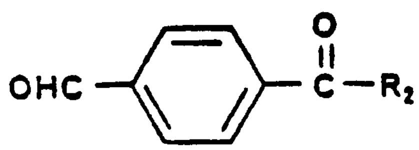

(III)

Ar, $R_1$ et $R_2$ ayant la signification donnée précédemment.

Cette réaction conduit généralement aux isomères de configuration E.

Les isomères Z peuvent être obtenus notamment par irradiation des isomères E par un rayonnement U.V.

Les sels de phosphonium de formule (II) sont obtenus selon un procédé connu à partir des aldéhydes et cétones correspondants de formule

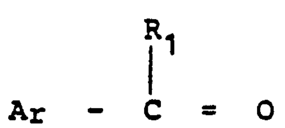

(IV)

Pour préparer les composés de formule (I), la technique préférée est d'obtenir par le procédé décrit ci-dessus, le composé de formule (I) dans lequel $R_2$ représente un groupe éthoxy, puis par hydrolyse, synthétiser l'acide correspondant ($R_2$ = OH); les esters et amides correspondantes

($R_2$ = alkoxy, $-OA_r$, $-O-(CH_2)_n$ - 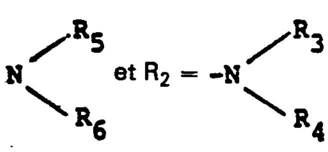 et $R_2$ = )

sont obtenus par des procédés courants connus de l'homme de l'art à partir de l'acide par l'intermédiaire du

chlorure d'acide ($R_2$ = Cl).

Les sels des composés de formule (I) peuvent être préparés de manière classique par réaction avec une base ou un acide, dans un solvant usuel.

On donnera ci-après dans le Tableau I des exemples de composés de formule I de configuration E.

Les exemples suivants illustrent la préparation de ces composés.

**EXEMPLE 1**

**Préparation du (E) - [(isopropyl-4 phényl)-2 propényl]-4 benzoate d'éthyle**

a) Préparation du bromure d'(isopropyl-4 phényl)-1 éthyl triphényl phosphonium.

A 0,32 mole de p.acétylcumène dissous dans 500 ml de méthanol, on ajoute à 0° C 0,17 mole de $NaBH_4$.

On agite 1 heure après l'addition et on verse le mélange réactionnel dans 2 litre d'HCl 1N glacé. On extrait avec de l'éther diéthylique, lave la phase organique avec une solution saturée de $NaHCO_3$, on la sèche et on évapore le solvant.

L'huile brute est aussitôt traitée à 0° C dans un mélange éther-hexane (10-100 v/v) contenant 5 gouttes de pyridine; 15 ml de $PBr_3$ sont alors ajoutés à la solution. On poursuit l'agitation pendant 2 h; on verse le mélange réactionnel dans l'eau glacée et extrait avec de l'éther diéthylique; la phase organique est lavée avec une solution saturée de $NaHCO_3$, puis séchée sur sulfate de sodium. Après évaporation du solvant, on ajoute 0,31 mole le triphényl phosphine à l'huile résiduelle dissoute dans 200 ml de xylène et on porte à 100° C pendant 48 heures. On essore le bromure d'(isopropyl-4 phényl)-1 éthyl triphényl phosphonium après refroidissement (Rendement: 76 %).

b) Préparation du (E) - [(isopropyl-4 phényl)-2 propényl)]-4 benzoate d'éthyle

On porte à reflux pendant 12 heures un mélange de 0,118 mole de bromure d'(isopropyl-4 phényl)-1 éthyl triphényl phosphonium, 0,129 mole de p.éthoxycarbonyl benzaldéhyde et 500 ml d'époxy-1,2 butane. On essore le précipité formé après refroidissement. On évapore sous vide le solvant du filtrat. L'huile résiduelle est triturée dans un mélange éther diéthylique-éther de pétrole (200-600 v/v). La fraction insoluble est éliminée par filtration. Le filtrat après évaporation est cristallisé dans 50 ml de méthanol froid.

Rendement : 41 %.

Cristaux blanc F = 65 - 67° C.

RMM ($CDCl_3$) : 1,26 ppm 6 H (d); 1,36 ppm 3 H (E);
2,26 ppm 3 H (d); 2,93 ppm 1 H (m);
4,40 ppm 2 H (q); 6,90 1 H (s.élargi);
7,20 à 8,20 ppm 8 H (m).

**EXEMPLE 2**

**Préparation de l'acide (E) - [(isopropyl-4 phényl)-2 propényl]-4 benzoïque**

On porte à reflux pendant 3 heures un mélange de 0,04 mole de (E) - [(isopropyl-4 phényl)-2 propényl]-4 benzoate d'éthyle, 0,1 mole de potasse, 200 ml d'éthanol et 60 ml d'eau distillée. Après refroidissement, on acidifie le mélange réactionnel avec de l'acide chlorhydrique. L'acide brut est séparé par filtration, puis recristallisé dans du méthanol.

Rendement : 90 %.

Cristaux blancs - F : 235 - 240° C

RMN ($COCl_3$) + ε DMSO) 1,25 ppm 6 H (d); 2,30 ppm 3 H (s.élargi);
2,86 ppm 1 H (m); 6,90 ppm 1 H (s.élargi);
7,16 à 8,16 ppm 8 H (m).

**EXEMPLE 3**

**Préparation de la (E) - [(isopropyl-4 phényl)-2 propényl]-4 N-benzoyl morpholine**

a) Préparation du chlorure de l'acide (E) - [isopropyl-4 phényl)-2 propényl)-4 benzoïque

On agite à température ambiante pendant 20 heures 0,01 mole d'acide correspondant en présence de 0,02 mole de chlorure de thionyle, 100 ml d'éther diéthylique, 1 ml de pyridine et 3 gouttes de diméthylfornamide. Après filtration, la solution éthérée est évaporée et le résidu trituré dans du benzène. Après évaporation sous vide du benzène, le chlorure d'acide est isolé et utilisé pour la suite sans autre purification.
Rendement : 84 %.

b) Préparation de la (E) - [(isopropyl-4 phényl)-2 propényl]-4 N-benzoyl morpholine.

On ajoute goutte à goutte à une solution de 0,01 mole de morpholine dans 20 ml de benzène maintenue à reflux, 0,05 mole de chlorure d'acide (E) - [(isopropyl-4 phényl)-2 propényl]-4 benzoïque dans 10 ml de benzène. Après 4 heures de reflux, la fraction insoluble est séparée par filtration, la solution benzénique est évaporée sous vide et le résidu rectristallisé dans un mélange éther diéthylique-éther de pétrole.
Rendement : 57 %.
Cristaux blancs - F : 100 - 102° C.

RMN ($CDCl_3$) : 1,28 ppm 6 H (d) ; 2,28 ppm 3 H (d);
2,95 ppm 1 H (m); 3,72 ppm 8 H (s.élargi);
6,85 ppm 1 H (s.élargi); 7,20 à 7,60 ppm 8H (m).

**EXEMPLE 4**

**Préparation du chlorhydrate de l'ester N,N-diéthylaminoéthylique de l'acide (E) - (isopropyl-4 phényl)-2 propényl]-4 benzoïque**

On ajoute goutte à goutte 0,003 mole de chlorure de l'acide (E) - [(isopropyl-4 phényl)-2 propényl]-4 benzoïque dans 20 ml de benzène à une solution de 0,006 mole de N,N-diéthylaminoéthanol dans 30 ml de benzène. Après 3 heures de reflux, la solution benzénique est filtrée, lavée avec de l'eau, puis séchée. Le solvant est évaporé sous vide et l'ester brut résiduel dissous dans l'éther éthylique est transformé en chlorhydrate par barbotage d'acide chlorhydrique gazeux. Le chlorhydrate de l'ester est ensuite recristallisé dans un mélange méthanol-éther diéthylique.
Rendement : 70 %.
Cristaux blancs - F : 170 - 172° C.

**TABLEAU I**

Composés de formule I

| Composé N° | Ar | $R_1$ | $R_2$ | Rendement (%) | F (°C) | Solvant de cristallisation |
|---|---|---|---|---|---|---|
| 1 | 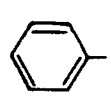 | $CH_3$ | -OH | 75 | 195 - 7 | Ethanol |
| 2 | " | $CH_3$ | $-O-CH_3$ | 40 | 65 - 7 | Méthanol |
| 3 | 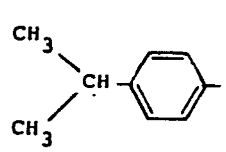 | $CH_3$ | -OH | 90 | 235 - 40 | Méthanol |
| 4 | " | $CH_3$ | $-NH_2$ | 84 | 204 - 6 | Ethanol |
| 5 | " | $CH_3$ | 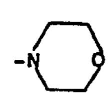 | 57 | 100 - 2 | Ether diéthylique-Ether de pétrole |

**TABLEAU I**

(suite)

| Composé N° | Ar | $R_1$ | $R_2$ | Rendement (%) | F (°C) | Solvant de cristallisation |
|---|---|---|---|---|---|---|
| 6 | 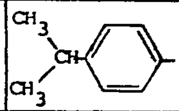 | $CH_3$ | $-O-CH_2-CH_3$ | 41 | 65 - 7 | Méthanol |
| 7 | " | $CH_3$ | 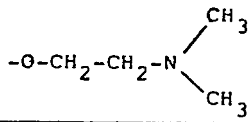 | 44 | Chlorhydrate 183 - 5 | Méthanol |
| 8 | " | $CH_3$ | 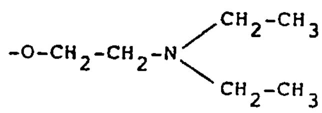 | 70 | Chlorhydrate 170 - 2 | Méthanol |
| 9 | 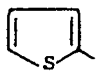 | $CH_3$ | -OH | 90 | 169 - 71 | Ethanol |
| 10 | " | $CH_3$ | $-O-CH_2-CH_3$ | 51 | 49 - 51 | Méthanol |

**TABLEAU I**

(suite)

| Composé N° | Ar | $R_1$ | $R_2$ | Rendement (%) | F (°C) | Solvant de cristallisation |
|---|---|---|---|---|---|---|
| 11 | 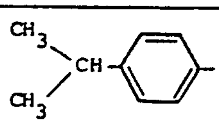 | $CH_3$ | 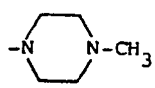 | 65 | chlorhydrate 247-249 | Méthanol |
| 12 | 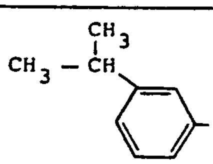 | $CH_3$ | - OH | 42 | 128-130 | Ethanol |
| 13 | 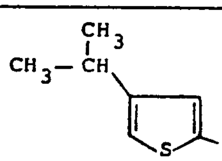 | $CH_3$ | - OH | 70 | 190-2 | Ethanol |
| 14 | 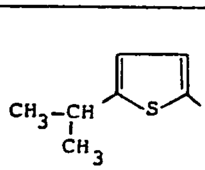 | $CH_3$ | - OH | 40 | 146-8 | Ethanol |

On donnera ci-après les résultats des études pharmacologiques mettant en évidence les propriétés des composés de formule I.

**Affinité pour le récepteur cytosolique de l'acide rétinoïque (C.R.A.B.P.) des testicules de rats**

L'affinité des composés de formule I pour le récepteur C.R.A.B.P. est déterminée par mesure de l'inhibition de la liaison spécifique de l'acide rétinoïque tritié (*AR) par les différents composés suivant la méthode de ONG et CHYTIL (J. Biol. Chem., 1975, 250, 6113) modifiée:

Les testicules de rats décongelés à 4°C sont placés dans du tampon Tris-HCl 50 mM, DTE 2 mM pH 7,4, puis broyés dans un broyeur Potter téflon/verre (trois fois à 800 t/mn). Le broyat est centrifugé à 4000 x g pendant 10 mn à 4°C.

Le surnageant est alors centrifugé 60 mn à 105000 x g à 4°C pour obtenir le cytosol. La concentration finale en protéines du cytosol est ajustée à 2 mg/ml.

200 µl de cytosol sont incubés pendant 16 h à 4°C en présence d'acide rétinoïque tritié 20 nM (activité spécifique: 30 Ci/mmole), et en présence des ligands à teser à la concentration de 4 µM.

Les incubations sont suivies d'un traitement au charbon-dextran (1 % - 0,0025 %) pendant 30 mn à 4°C. La radioactivité liée est déterminée par comptage en scintillation liquide.

Le Tableau II ci-après rapporte les pourcentages d'inhibition de liaison de l'acide rétinolque tritié pour les composés de formule I possédant un groupe -COOH ($R_2$ = OH), l'inhibition de la liaison avec de l'acide rétinoïque non marqué correspondant à 100 % d'inhibition. Ce tableau met en évidence l'affinité de ces composés pour le récepteur C.R.A.B.P.

Les composés de formule 5 qui ne possèdent pas de groupe acide libre ne lient pas ou peu le récepteur C.R.A.B.P. et ne sont donc pas ou ne sont que peu actifs dans ce test, mais ont cependant une activité pharmacologique analogue, car ils constituent vraisemblablement des précurseurs (prodrugs) des composés à groupe acide.

**TABLEAU II**

| Composé n° | % d'inhibition de la liasion au récepteur C.R.A.B.P. |
|---|---|
| Acide rétioïque | 100 |
| 1 | 18,9 |
| 3 | 68,5 |
| 9 | 18,5 |
| 11 | 27,5 |
| 12 | 67,5 |
| 13 | 72,5 |
| 14 | 75,0 |

Les compositions thérapeutiques selon l'invention peuvent être administées à l'homme ou aux animaux par voie topique, orale ou parentérale.

Elles peuvent être sous la forme de prépartions solides, semi-solides ou liquides. Comme exemple, on peut citer les comprimés, les gélules, les suppositoires, les solutions ou suspensions injectables, les pommades, les collyres, les colutoires, ainsi que les formes-retard et les formes implantées à libération lente.

Dans ces compositions, le principe actif est généralement mélange avec un ou plusieurs bien connus de l'homme de l'art.

Les compositions thérapeutiques administrables par voie topique peuvent contenir notamment de 0,001 à 5 % en poids de principe actif.

La quantité de principe actif administrée dépend évidemment du patient qui est traité, de la voie d'administration et de la sévérité de la maladie.

**Revendications** pour les Etats Contractants: BE, CH, DE, FR, GB, IT, LI, LN, NL, SE

1. Composés de formule

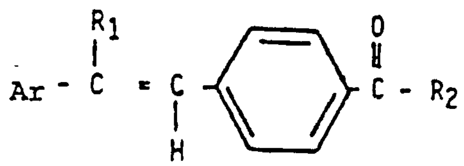

(I)

dans laquelle:

$R_1$ représente un groupe méthyle;

Ar raprésente un groupe phényle, un groupe phényle substitué par un groupe alkyle en $C_1$-$C_4$, un groupe hétéroaromatique monocyclique contentant un ou deux hétéroatomes choisis parmi l'azote, l'oxygène et le soufre et ayant de 5 à 11 chaînons ou un tel groupe hétéroarmatique substitué par un groupe alkyle en $C_1$-$C_4$;

$R_2$ réprésente un groupe hydroxy; un groupe aminé de formule

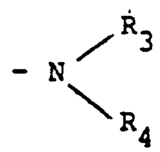

dans laquelle $R_3$ et $R_4$ représentent, indépendamment l'un de l'autre, un atome d'hydrogène, un groupe alkyle en $C_1$-$C_6$ ou amino(alkyle en $C_1$-$C_6$) ou bien $R_3$ et $R_4$ forment, avec l'atome d'azote sur lequelle ils sont fixés, un groupe hétérocyclique saturé ou ayant de 5 à 8 chaînons et pouvant contenir un autre hétéroatome choisi parmi l'azote, l'oxygène et le soufre; un groupe alkoxy en $C_1$-$C_4$; un groupe de formule -O-Ar, Ar ayant la signification donnée ci-dessus; un groupe aminoalkoxy de formule

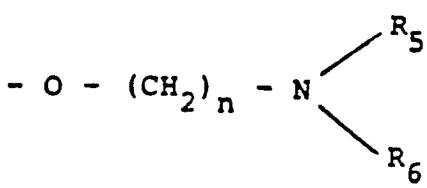

dans laquelle $R_5$ et $R_6$ représentent, indépendamment l'un de l'autre, un groupe alkyle en $C_1$-$C_4$ et n = 1 à 4; ainsi que leurs sels pharmaceutiquement acceptables.

2. Composés selon la revendication 1 ayant la configuration E.

3. Composés selon la revendication 2 dans lesquels $R^2$ est un groupe OH.

4. Composés selon la revendication 2 ou la revendication 3 dans lesquels Ar est un groupe phényle ou thiényle substitué par un groupe isopropyle.

5. Procédé de préparation d'un composé selon la revendication 1, caractérisé en ce que l'on fait réagir un composé de formule

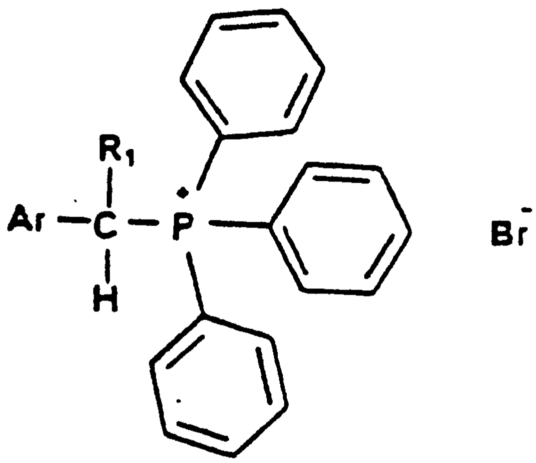

(II)

avec un benzaldéhyde de formule

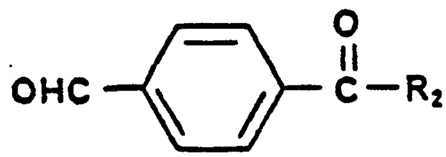

(III)

Ar, $R_1$ et $R_2$ ayant la signification donnée précédemment.

6. Procédé selon la revendication 5, caractérisé en ce que l'on prépare par ledit procédé un composé de formule I dans laquelle $R_2$ est un groupe éthoxy, puis on effectue une hydrolyse pour obtenir un composé de formule I dans laquelle $R_2$ est un groupe hydroxy, et éventuellement on transforme l'acide en les esters et amides correspondants.

7. Composition thérapeutique qui contient à titre de principe actif un composé selon l'une quelconque des revendications 1 à 4.

8. Composition selon la revendication 7, caractérisée en ce qu'elle est sous une forme administrable par voie topique.

9. Utilisation d'un composé de formule I telle que définie à la revendication 1 et dans laquelle $R_2$ est un groupe hydroxy ou d'un de ses sels comme ligand du récepteur C.R.A.B.P.

10. Réactif biochimique comprenant des récepteurs C.R.A.B.P. liés à un composé de formule I telle que définie à la revendication 1 et dans laquelle $R_2$ est un groupe hydroxy ou à l'un de ses sels.

**Revendications** pour l'Etat Contractant: AT

1. Procédé de préparation d'un composé de formule

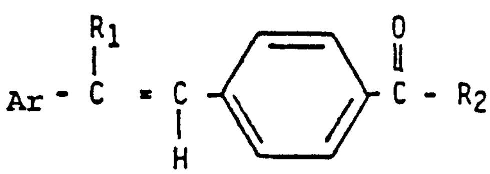

(I)

dans laquelle:

$R^1$ représente un groupe méthyle;

Ar représente un groupe phényle, un groupe phényle substitué par un groupe alkyle en $C_1$-$C_4$, un groupe hétéroaromatique monocyclique contenant un ou deux hétéroatomes choisis parmi l'azote, l'oxygène et le soufre et ayant de 5 à 11 chaînons ou un tel groupe hétéroaromatique substitué par un groupe alkyle en $C_1$-$C_4$;

$R_2$ représente un groupe hydroxy; un groupe aminé de formule

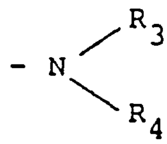

dans laquelle $R_3$ et $R_4$ représentent, indépendamment l'un de l'autre, un atome d'hydrogène, un groupe alkyle en $C_1$-$C_6$ ou amino(alkyle en $C_1$-$C_6$) ou bien $R_3$ et $R_4$ forment, avec l'atome d'azote sur lequel ils sont fixés, un groupe hétérocyclique saturé ou non ayant de 5 à 8 chaînons et pouvant contenir un autre hétéroatome choisi parmi l'azote, l'oxygène et le soufre; un groupe alkoxy en $C_1$-$C_4$; un groupe de formule -O-Ar, Ar ayant la signification donnée ci-dessus; un groupe aminoalkoxy de formule

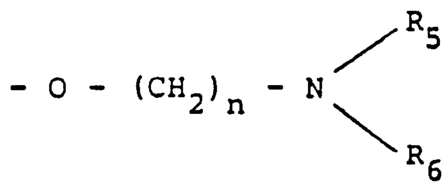

dans laquelle $R_5$ et $R_6$ représentent, indépendamment l'un de l'autre, un groupe alkyle en $C_1$-$C_4$ et n = 1 à 4; ainsi que ses sels pharmaceutiquement acceptables, caractérisé en ce que l'on fait réagir un composé de formule

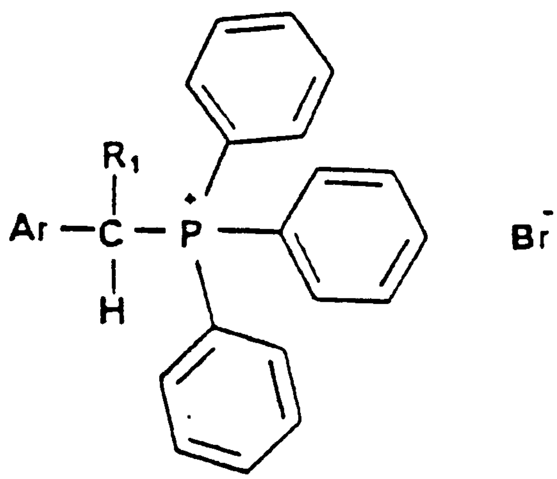

(II)

avec un benzaldéhyde de formule

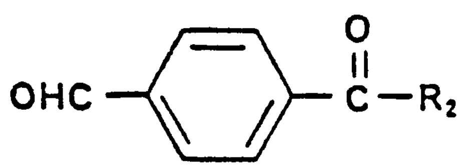

(III)

Ar, $R^1$ et $R^2$ ayant la signification donnée précédemment.

2. Procédé selon la revendication 1, caractérisé en ce que l'on prépare par ledit procédé un composé de formule I dans laquelle $R_2$ est un groupe éthoxy, puis on effectue une hydrolyse pour obtenir un composé de formule I dans laquelle $R_2$ est un groupe hydroxy, et éventuellement on transforme l'acide en les esters et amides correspondants.

3. Procédé de préparation d'une composition thérapeutique, caractérisé en ce que l'on mélange un composé de formule I telle que définie à la revendication 1 ou l'un de ses sels pharmaceutiquement acceptables avec un excipient pharmaceutiquement acceptable.

4. Utilisation d'un composé de frmule I telle que définie à la revendication 1 et dans laquelle $R^2$ est un groupe hydroxy ou d'un de ses sels comme ligand du récepteur C.R.A.B.P.

5. Réactif biochimique comprenant des récepteurs C.R.A.B.P. liés à un composé de formule I telle que définie à la revendication 1 et dans laquelle $R^2$ est un groupe hydroxy ou à l'un de ses sels.

**Patentansprüche** für die Vertragsstaaten: BE, CH, DE, FR, GB, IT, LI, LU, NL, SE

1. Verbindungen der Formel

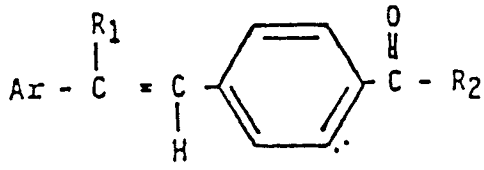

(I)

in der $R_1$ ein Methylrest ist, Ar für einen Phenylrest, einen mit einem $C_1$-$C_4$-Alhylrest substituierten Phenylrest, eine heteroaromatische monocyclische Gruppe, die ein oder zwei aus Stickstoff, Sauerstoff und Schwefel ausgewählte Heteroatome enthält und 5 bis 11 Kettenglieder aufweist, oder eine solche heteroaromatische Gruppe, die mit einem $C_1$-$C_4$-Alkylrest substituiert ist, steht,

$R_2$ eine Hydroxylgruppe, eine Aminogruppe der Formel

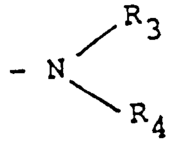

in der $R_3$ und $R_4$ unabhängig voneinander ein Wasserstoffatom, ein $C_1$-$C_6$-Alkylrest oder eine Amino($C_1$-$C_6$-alkyl)-gruppe sind oder $R_3$ und $R_4$ mit dem Stickstoffatom, an das sie gebunden sind, eine gesättigte oder ungesättigte heterocyclische Gruppe bilden, die 5 bis 8 Kettenglieder aufweist und ein weiteres, aus Stickstoff, Sauerstoff und Schwefel ausgewähltes Heteroatom enthalten kann, ein $C_1$-$C_4$-Alkoxyrest, eine Gruppe der Formel -O-Ar, worin Ar die vorstehend genannte Bedeutung hat, eine Aminoalkoxygruppe der Formel

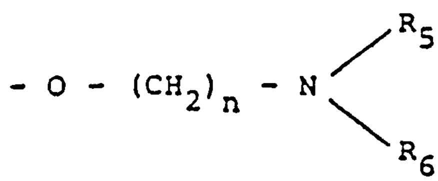

in der $R_5$ und $R_6$ unabhängig voneinander ein $C_1$-$C_4$-Alkylrest sind, ist und n einen Wert von 1 bis 4 hat, sowie ihre pharmazeutisch unbedenklichen Salze.

2. Verbindungen nach Anspruch 1 mit der Konfiguration E.

3. Verbindungen nach Anspruch 2, in denen $R_2$ eine OH-Gruppe ist.

4. Verbindungen nach Anspruch 2 oder Anspruch 3, in denen Ar ein mit einer Isopropylgruppe substituierter Phenyl- oder Thienylrest ist.

5. Verfahren zur Herstellung einer Verbindung nach Anspruch 1, dadurch gekennzeichnet, daß man eine Verbindung der Formel

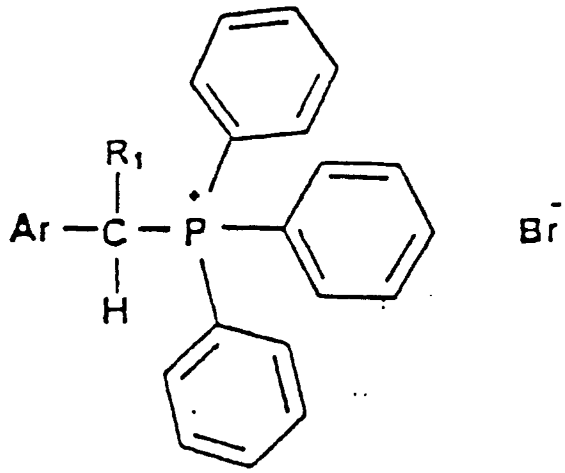

(II)

mit einem Benzaldehyd der Formel

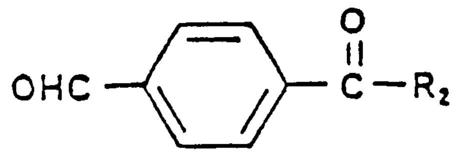

(III)

wobei Ar, $R_1$ und $R_2$ die vorstehend genannten Bedeutungen haben, umsetzt.

6. Verfahren nach Anspruch 5, dadurch gekennzeichnet, daß man nach dem genannten Verfahren eine Verbindung der Formel I herstellt, in der $R_2$ eine Ethoxygruppe ist, dann eine Hydrolyse durchführt, um eine Verbindung der Formel I, in der $R_2$ eine Hydroxylgruppe ist, zu erhalten und gegebenenfalls die Säure in die Ester und entsprechenden Amide umwandelt.

7. Therapeutische Zubereitung, die als Wirkstoff eine Verbindung nach irgendeinem der Ansprüche 1 bis 4 enthält.

8. Zubereitung nach Anspruch 7, dadurch gekennzeichnet, daß sie in einer auf örtlichem Wege verabreichbaren Form vorliegt.

9. Verwendung einer Verbindung der Formel I, wie sie in Anspruch 1 definiert wurde und in der $R_2$ eine Hydroxylgruppe ist, oder eines ihrer Salze als Ligand des C.R.A.B.P.-Rezeptors.

10. Biochemisches Reagenz, umfassend C.P.A.B.P.-Rezeptoren, die an eine Verbindung der Formel I, wie sie in Anspruch 1 definiert wurde und in der $R_2$ eine Hydroxylgruppe ist, oder eines ihrer Salze gebunden sind.

**Patentansprüche** für den Vertragsstaat: AT

1. Verfahren zur Herstellung einer Verbindung der Formel

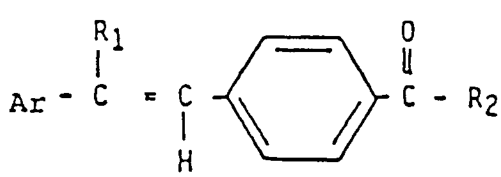

(I)

in der $R_1$ ein Methylrest ist, Ar für einen Phenylrest, einen mit einem $C_1$-$C_4$-Alkylrest substituierten Phenylrest, eine heteroaromatische monocyclische Gruppe, die ein oder zwei aus Stickstoff, Sauerstoff und Schwefel ausgewählte Heteroatome enthält und 5 bis 11 Kettenglieder aufweist, oder eine solche heteroaromatische Gruppe, die mit einem $C_1$-$C_4$-Alkylrest substituiert ist, steht,

$R_2$ eine Hydroxylgruppe,eine Aminogruppe der Formel

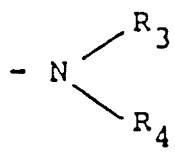

in der $R_3$ und $R_4$ unabhängig voneinander ein Wasserstoffatom, ein $C_1$-$C_6$-Alkylrest oder eine Amino($C_1$-$C_6$-alkyl)-gruppe sind oder $R_3$ und $R_4$ mit dem Stickstoffatom, an das sie gebunden sind, eine gesättigte oder ungesättigte heterocyclische Gruppe bilden, die 5 bis 8 Kettenglieder aufweist und ein weiteres, aus Stickstoff, Sauerstoff und Schwefel ausgewähltes Heteroatom enthalten kann, ein $C_1$-$C_4$-Alkoxyrest, eine Gruppe der Formel -O-Ar, worin Ar die vorstehend genannte Bedeutung hat, eine Aminoalkoxygruppe der Formel

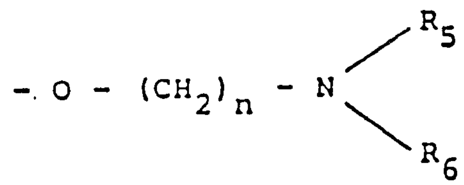

in der $R_5$ und $R_6$ unabhängig voneinander ein $C_1$-$C_4$-Alkylrest sind, ist und n einen Wert von 1 bis 4 hat, sowie ihrer pharmazeutisch unbedenklichen Salze, dadurch gekennzeichnet, daß man eine Verbindung der Formel

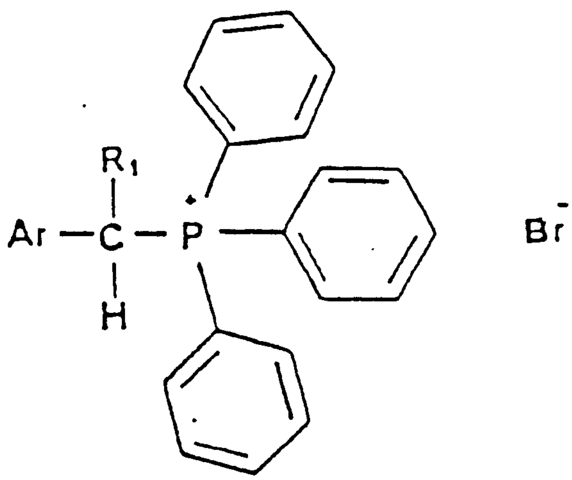

(II)

mit einem Benzaldehyd der Formel

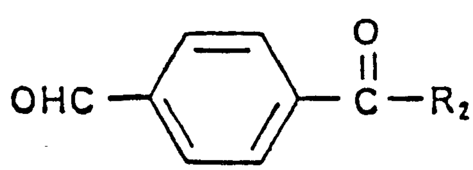

(III)

wobei Ar, $R_1$ und $R_2$ die vorstehend genannten Bedeutungen haben, umsetzt.

2. Verfahren nach Anspruch 1, dadurch gekennzeichnet, daß man nach dem genannten Verfahren eine Verbindung der Formel I herstellt, in der $R_2$ ein Ethoxyrest ist, dann eine Hydrolyse durchführt, um eine Verbindung der Formel I zu erhalten, in der $R_2$ eine Hydroxylgruppe ist, und gegebenenfalls die Saure in die Ester und entsprechenden Amide umwandelt.

3. Verfahren zur Herstellung einer therapeutischen Zubereitung, dadurch gekennzeichnet, daß man eine Verbindung der Formel I, wie sie in Anspruch 1 definiert wurde, oder eines ihrer pharmazeutisch unbedenklichen Salze mit einem Pharmazeutisch unbedenklichen Hilfsstoff mischt,

4. Verwendung einer Verbindung der Formel I, wie sie in Anspruch 1 definiert wurde und in der $R_2$ eine Hydroxylgruppe ist, oder eines ihrer Salze als Ligand des C.R.A.B.P.-Rezeptors

5. Biochemisches Reagenz, umfassend C.R.A.B.P.-Rezeptoren, die an eine Verbindung der Formel I, wie sie in Anspruch 1 definiert wurde und in der $R_2$ eine Hydroxylgruppe ist, oder an eines ihrer Salze gebunden sind.

**Claims** for the contracting states: BE, CH, DE, FR, GB, IT, LI, LU, NL, SE

1. Compounds of the formula

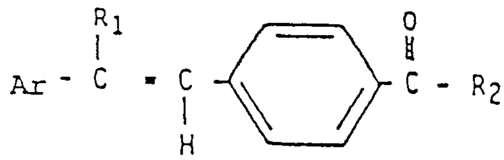

(I)

in which:

$R_1$ represents a methyl group;

Ar represents a phenyl group, a phenyl group substituted by a $C_1$-$C_4$ alkyl group, a 5 - 11 membered monocyclic heteroaromatic group containing one or two heteroatoms selected from nitrogen, oxygen and sulfur or such a heteroaromaticgroup substituted by a $C_1$-$C_4$ alkyl group;

$R_2$ represents a hydroxy group; an amino group of the formula

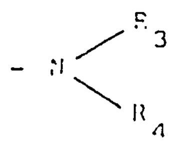

in which $R_3$ and $R_4$ represent, independently from one another, a hydrogen atom, a $C_1$-$C_6$ alkyl group or amino ($C_1$-$C_6$)alkyl or $R_3$ and $R_4$ form, with the nitrogen atom to which they are attached, a 5 - 8 membered heterocyclic group saturated or not and which can contain another heteroatom selected from nitrogen, oxygen and sulfur; $C_1$-$C_4$ alkoxy group; a group of the formula -O-Ar, Ar having the above-given meaning; an aminoalkoxy group of the formula

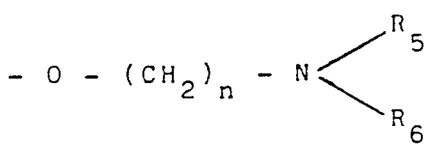

in which $R_5$ and $R_6$ represent, independently from one another a $C_1$-$C_4$ alkyl group and n = 1 to 4; and their pharnaceutically acceptable salts.

2. Compounds according to claim 1, having the E configuration.

3. Compounds according to claim 2 wherein $R_2$ is an OH group.

4. Compounds according to claim 2 or claim 3 in which Ar is a phenyl or thienyl group substituted by an isopropyl group.

5. Process for preparing a compound according to claim 1, wherein a compound of the formula

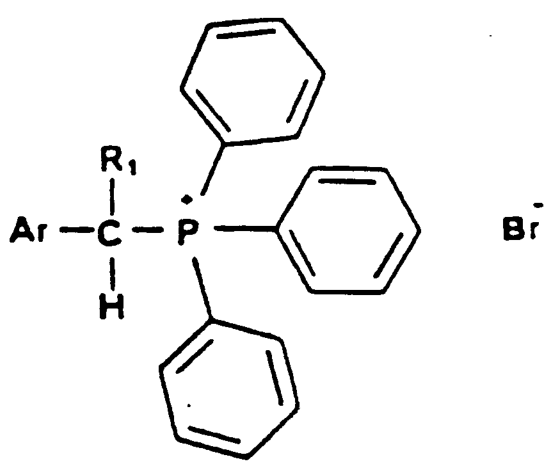

(II)

is reacted with a benzaldehyde of the formula

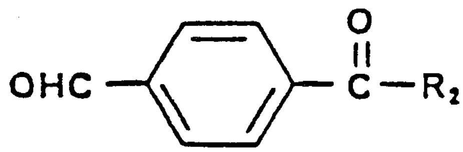 (III)

Ar, $R_1$ and $R_2$ having the previously given meaning.

6. Process according to claim 5, wherein a compound of formula I in which $R_2$ is an ethoxy group, is prepared by said process, then hydrolysis is carried out to obtain a compound of formula I in which $R_2$ is a hydroxy group, and optionally the acid is converted into the corresponding esters and amides.

7. Therapeutic composition which comprises as active ingredient a compound according to any one of claims 1 to 4.

8. Composition according to claim 7, in a topically administrable form.

9. Use of the compound of the formula I as defined in claim 1 and in which $R_2$ is a hydroxy group or one of its salts as a ligand of the C.R.A.B.P. receptor.

10. Biochemical reagent comprising C.R.A.B.P. receptors linked to a compound of the formula I such as defined in claim 1 and in which $R_2$ is a hydroxy group or one of its salts.

**Claims** for the contracting state: AT

1. Process for the preparation of a compound of the formula

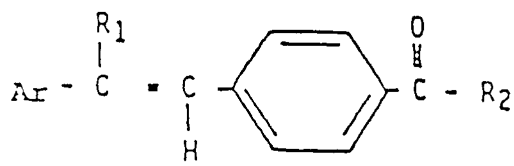 (I)

in which

$R_1$ represents a methyl group;

Ar represents a phenyl group, a phenyl group substituted by a $C_1$-$C_4$ alhyl group, a 5 - 11 membered monocyclic heteroaromatic group containing one or two heteroatoms selected from nitrogen, oxygen and sulfur or such a heteroaromaticgroup substituted by a $C_1$-$C_4$ alkyl group;

$R_2$ represents a hydroxy group; an amino group of the formula

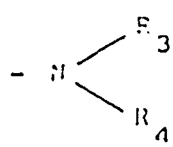

in which $R_3$ and $R_4$ represent, independently from one another, a hydrogen atom, a $C_1$-$C_6$ alkyl group or amino $(C_1$-$C_6)$alkyl or $R_3$ and $R_4$ form, with the nitrogen atom to which they are attached, a 5 - 8 membered heterocyclic group saturated or not and which can contain another heteroatom selected from nitrogen, oxygen and sulfur; $C_1$-$C_4$ alkoxy group; a group of the formula -O-Ar, Ar having the above-given meaning; an aminoalkoxy group of the formula

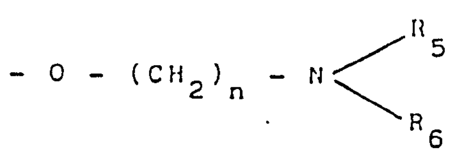

in which $R_5$ and $R_6$ represent, independently from one another a $C_1$-$C_4$ alkyl group and n = 1 to 4; and of their pharmaceutically acceptable salts, characterized in that a compound of the formula

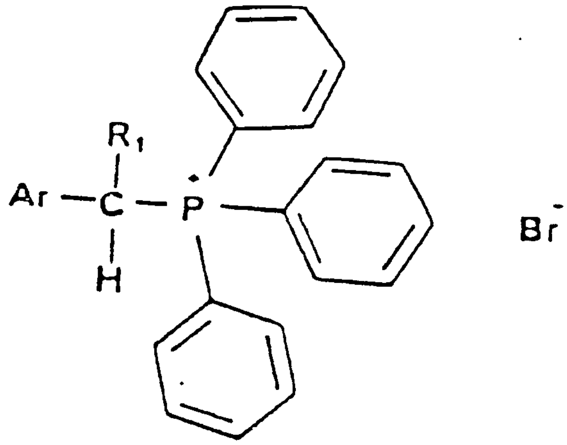

(II)

is reacted with a benzaldehyde of the formula

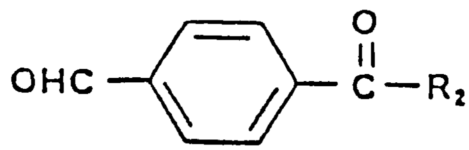

(III)

Ar, $R_1$ and $R_2$ having the previously given meaning.

2. Process according to claim 1, characterized in that a compound of formula I in which $R_2$ is an ethoxy group, is prepared by said process, then hydrolysis is carried out to obtain a compound of formula I in which $R_2$ is a hydroxy group, and optionally the acid is converted into the corresponding esters and amides.

3. Process for the preparation of a therapeutic composition, characterized in that a compound of the formula I such as defined in claim 1 or one of its pharmaceutically acceptable salts is mixed with a pharmaceutically acceptable carrier.

4. Use of the compound of the formula I as defined in claim 1 and in which $R_2$ is a hydroxy group or one of its salts as a ligand of the C.R.A.B.P. receptor.

5. Biochemical reagent comprising C.R.A.B.P. receptors linked to a compound of the formula I such as defined in claim 1 and in which $R_2$ is a hydroxy group or one of its salts.